(19) Europäisches Patentamt
European Patent Office
Office européen des brevets

(11) **EP 2 433 565 B1**

(12) **EUROPÄISCHE PATENTSCHRIFT**

(45) Veröffentlichungstag und Bekanntmachung des Hinweises auf die Patenterteilung:
**03.06.2015 Patentblatt 2015/23**

(51) Int Cl.:
*A61B 5/00* *(2006.01)*      *A61N 1/368* *(2006.01)*
*A61B 5/11* *(2006.01)*      *A61N 1/365* *(2006.01)*

(21) Anmeldenummer: **11179133.1**

(22) Anmeldetag: **29.08.2011**

(54) **Implantierbares medizinischen Gerät**

Implantable medical device

Appareil médical implantable

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.09.2010 US 387022 P**

(43) Veröffentlichungstag der Anmeldung:
**28.03.2012 Patentblatt 2012/13**

(73) Patentinhaber: BIOTRONIK SE & Co. KG
**12359 Berlin (DE)**

(72) Erfinder:
• **Skerl, Olaf**
**18209 Bad Doberan (DE)**
• **Lippert, Michael**
**91522 Ansbach (DE)**
• **Kirchner, Jens**
**91052 Erlangen (DE)**

(74) Vertreter: **Lindner-Vogt, Karin L.**
**Biotronik GmbH & Co. KG**
**Corporate Intellectual Properties**
**Woermannkehre 1**
**12359 Berlin (DE)**

(56) Entgegenhaltungen:
US-A- 5 836 987      US-A1- 2002 173 826
US-A1- 2008 194 975      US-A1- 2008 243 202
US-A1- 2009 138 060

EP 2 433 565 B1

**Beschreibung**

Die Erfindung betrifft ein implantierbares medizinisches Gerät, wie beispielsweise einen implantierbaren Herzschrittmacher oder Herzmonitor, mit Mitteln zum Erfassen eines Schlagvolumens eines Herzens.

Zur Bestimmung oder Abschätzung des Schlagvolumens (der Pumpleistung des Herzens) ist eine Vielzahl von Verfahren bekannt. Am weitesten verbreitet sind (vorrangig thermische) Dilutionsverfahren. Nachteilig bei den thermischen Dilutionsverfahren für die Verwendung in Implantaten ist ihr hoher Energieverbrauch oder die Notwendigkeit eines Zugangs von außerhalb des Körpers zum Einbringen eines Temperaturimpulses. Weiterhin sind kontinuierliche Messungen mit Dilutionsverfahren nur sehr eingeschränkt möglich. Bekannt sind auch Verfahren, die auf der Messung des Blutflusses basieren. Die Anordnungen zur Blutflussmessung mit implantierten Sensoren sind allerdings technisch relativ aufwendig oder weisen einen hohen Energiebedarf für die Messung oder die Verarbeitung der Sensorsignale auf.

Weitere Verfahren verwenden intrakardiale oder intrathorakale Impedanzsignale für die Ableitung von Parametern, die als Maß für das Schlagvolumen oder dessen Änderungen verwendet werden (z.B. US 7,395,114, EP 1 762 270, US 7,702,389). Bei den impedanzbasierten Verfahren werden die Parameter aus Leitfähigkeitsänderungen bestimmt, die nur mittelbar mit dem Schlagvolumen verbunden und von weiteren Faktoren beeinflusst sind. In US 7,395,114 wird beispielsweise aus der intrakardialen Impedanz ein Maß für die Ventrikelkontraktion abgeleitet und von diesem auf das Schlagvolumen geschlossen.

Mit Hilfe der Echokardiografie können ebenfalls Maße für das Schlagvolumen ermittelt werden. So können geometrische Änderungen des linken Ventrikels bestimmt und daraus ein Maß für das Schlagvolumen abgeleitet werden. Weiterhin kann über den DopplerEffekt der Blutfluss im Aortenbogen näherungsweise gemessen und daraus das Schlagvolumen bestimmt werden. Für Implantate sind diese Verfahren aber in der Regel zu aufwendig.

Als Alternative für Implantate wurden auch Verfahren entwickelt, bei denen aus den Herztönen Parameter abgeleitet werden, die unter bestimmten Annahmen mit dem Schlagvolumen korrelieren. Häufig werden hierbei Beschleunigungssensoren für die Aufnahme der Herztöne verwendet.

In US 7,139,609 werden die Herztöne aus dem Signal eines Beschleunigungssensors herausgelöst, beispielsweise durch geeignete Filterung. Aus diesen Herztönen, vorrangig aus dem ersten Herzton S1, werden anhand empirischer Zusammenhänge Parameter abgeleitet, die unter bestimmten Bedingungen in Zusammenhang mit dem Schlagvolumen stehen. Die Herztöne werden zusätzlich hinsichtlich Merkmalen analysiert, die auf eine gestörte Herzklappenfunktion hinweisen, um gegebenenfalls den berechneten Parameter eingeschränkt zu korrigieren. Weiterhin wird das Intrakardiale Elektrogramm (IEGM) in die Bestimmung der Parameter mit einbezogen, wodurch eine zusätzliche Elektrode notwendig wird. Da eine Vielzahl von weiteren Faktoren die Charakteristik der Herztöne beeinflussen, liegt es nahe, dass die Bestimmung der Parameter aus den Herztönen und deren Zusammenhang mit dem Schlagvolumen wenig zuverlässig ist. Ein ähnliches Verfahren wird beispielsweise in US 7,585,279 vorgestellt, bei dem ebenfalls aus dem Herzton S1 ein Maß für das Schlagvolumen approximiert wird.

Aus den Signalen eines Beschleunigungssensors im Implantat können bekanntermaßen auch andere Komponenten durch geeignete Signalverarbeitung herausgelöst werden. Eine dieser Komponenten ist das sog. Seismokardiogramm (SCG), das die Vibrationssignale darstellt, die durch das Schlagen des Herzens im Thorax entstehen. Das SCG enthält die durch die mechanische Bewegung des Herzens entstehenden Beschleunigungssignale im Thorax senkrecht zur Körperachse. Auch daraus lassen sich Parameter bestimmen, die unter bestimmten Bedingungen näherungsweise mit dem Schlagvolumen zusammenhängen, beispielsweise nach US 6,978,184. Dabei wird aber ein definierter und fester Zusammenhang zwischen der mechanischen Bewegung des Herzens und der Pumpleistung zugrunde gelegt, was besonders bei Herzinsuffizienz nicht uneingeschränkt gegeben ist.

Ein implantierbares medizinisches Gerät gemäss Oberbegriff des Anspruchs 1 ist aus US2009/0138060 A1 bekannt.

Der Erfindung liegt die Aufgabe zugrunde, ein alternatives implantierbares medizinisches Gerät mit Mitteln zum Erfassen eines Schlagvolumens eines Herzens zu schaffen. Erfindungsgemäß wird diese Aufgabe durch ein implantierbares medizinisches gemäss Anspruch 1 gelöst. Erfindungsgemäss weist das Gerät einen integrierten Beschleunigungssensor auf oder ist an einen ebenfalls implantierbaren Beschleunigungssensor anzuschließen. Der Beschleunigungssensor ist mit einer BCG-Erfassungseinheit verbundenen oder zu verbinden. Die BCG-Erfassungseinheit ist ausgebildet, ein von dem Beschleunigungssensor stammendes Beschleunigungssignal zu verarbeiten und aus dem 3D-Akzelerometer-Ausgangssignal ein Ballistokardiogramm (BCG) abzuleiten und an eine mit der BCG-Erfassungseinheit verbundene BCG-Auswerteeinheit auszugeben, die ausgebildet ist ein von der BCG-Erfassungseinheit stammendes Ballistokardiogramm dahingehend auszuwerten, dass die BCG-Auswerteeinheit ein ein jeweiliges Schlagvolumen repräsentierendes Ausgangssignal liefert

Die Erfindung schließt die Erkenntnis ein, dass ein Ballistokardiogramm (BCG) eine Signalkomponente des Beschleunigungssensors ist, welche die Beschleunigungssignale parallel zur Körperachse darstellt. Die Beschleunigungskomponente parallel zur Körperachse wird größtenteils durch die "Rückstoßwirkung" des ausgetriebenen Blutes hervorgerufen. In US 7,660,632 wird dieser bekannte Effekt aufgegriffen und als eine Möglichkeit zur Bestimmung der Herzfrequenz für die Steuerung eines implantierbaren Neurostimulators angewendet. Eine weitergehende Analyse des Ballistogramms wird nicht vorgenommen, und der Zusammenhang mit dem Schlagvolumen und einer Herzinsuffizienz wird darin nicht hergestellt. Erfindungsgemäss ist die BCG-Auswerteeinheit so ausgebildet, dass in einem von der BCG-Erfassungseinheit stammenden Ballistokardiogramm I- und J-Wellen bestimmt werden und aus der Amplitudendifferenz jeweils aufeinanderfolgender I- und J-Wellen das ein jeweiliges Schlagvolumen repräsentierende Ausgangssignal zu bilden.

Die Erfindung bietet den Vorteil, dass das Ballistokardiogramm (BCG) unmittelbar durch den Blutauswurf verursacht ist und damit ein direktes Maß für die Pumpleistung des Herzens liefert. Damit ist ein direktes Monitoring der Pumpleistung und des HF-Status möglich.

Das Ballistokardiogramm (BCG) stellt das Beschleunigungssignal parallel zur Körperachse dar. Diese Beschleunigung wird maßgeblich von der "Rückstoßwirkung" des aus dem linken Ventrikel ausgetriebenen Blutes verursacht. Es wird, ähnlich dem Elektrokardiogramm (EKG), in verschiedene Wellen unterteilt, die wesentlichen sind I, J, K, L und M. Im Gegensatz zum EKG, das die elektrische Erregung des Herzens darstellt, repräsentiert das BCG die hämodynamische Wirkung der Ventrikelkontraktion. Die J-Welle im BCG entsteht, wenn das Blut nach dem Öffnen der Aortenklappe aus dem linken Ventrikel in den Aortenbogen gedrückt wird. Der Beschleunigungsimpuls des ausströmenden Blutes (Blutimpuls) bewirkt einen entsprechenden Gegenimpuls ("Rückstoß") im Körper (Körperimpuls):

$$m_K \cdot a_K = -m_B \cdot a_B$$

Die Größe des Körperimpulses $a_K$ hängt neben der Körpermasse $m_K$ direkt ab von der Masse des ausgeworfenen Blutes $m_B$ und dessen Beschleunigung $a_B$. Damit können aus dem Körperimpuls $a_K$ Maße für die Menge (Masse) des ausgeworfenen Blutes und der Kontraktilität des Ventrikels abgeleitet werden. Aus diesen Maßen kann ein Parameter bestimmt werden, der in guter Näherung mit dem Schlagvolumen korreliert. Vorteilhaft ist, dass das BCG direkt mit der Pumpleistung des Herzens verbunden ist. In der Literatur werden beispielsweise bereits Verfahren beschrieben, mit denen ein Maß für das Schlagvolumen mit Hilfe einer modifizierten Personenwaage bestimmt werden kann [M. Etemadi, O. T. Inan, R. M. Wiard, G. T. A. Kovacs, and L. Giovangrandi, "Non-invasive assessment of cardiac contractility on a weighing scale", IEEE EMBC 2009, pp. 6773-6776, 2009].

Implantierte Schrittmacher/ICDs sind allgemein bereits mit einem Beschleunigungssensor ausgerüstet, um die Patientenaktivität zu erfassen und danach die Herzrate zu adaptieren. Bereits mit demselben Beschleunigungssensor lässt sich erfindungsgemäß ein Maß für das Schlagvolumen und die Kontraktilität bestimmen. Günstiger im Sinne der Erfindung wäre aber die Anwendung eines 3-achsigen Beschleunigungssensors.

Dementsprechend kann das BCG gemäß einer Ausführungsvariante der Erfindung mit einem bereits in dem implantierbaren medizinischen Gerät vorhandenen Beschleunigungssensor rein durch Anpassung der Signalverarbeitung bestimmt werden, vorteilhafter ist allerdings die Verwendung 3-achsiger Beschleunigungssensoren.

Eine vorteilhafte Ausführungsvariante des implantierbaren medizinischen Gerätes (IMD), welches beispielsweise ein Herzschrittmacher, ICD, Monitoringimplantat, Drucksensorimplantat oder ähnliches sein kann, enthält somit einen geeigneten, bevorzugt 3-achsigen, Beschleunigungssensor. Das implantierbare medizinische Gerät kann zusätzlich Elektroden zur Aufnahme der elektrischen Aktivität des Herzens (EKG, IEGM) enthalten, sowie auch weitere Sensoren (z.B. Drucksensoren).

Eine 3-achsige Ausführung des Beschleunigungssensors ermöglicht die Bestimmung der räumlichen Lage der einzelnen Beschleunigungsvektoren. Dadurch kann zum einen die Ausrichtung des implantierbaren medizinischen Gerätes im Körper kompensiert werden und andererseits die Beschleunigungsvektoren senkrecht und parallel zur Körperachse getrennt bestimmt werden.

Aus den Signalen des Beschleunigungssensors wird die parallel zur Körperachse liegende Komponente ermittelt. Aus dem Beschleunigungssignal parallel zur Körperachse wird durch geeignete Methoden das Ballistokardiogramm herausgelöst, beispielweise durch geeignete Bandpass-Filter, die die Herztöne und die Bewegungsanteile unterdrücken. Das BCG-Signal kann zur Verbesserung der Signalqualität geeignet weiterverarbeitet werden, beispielsweise durch synchrone Mittelung über mehrere Herzzyklen oder zusätzliche Filterung zur Unterdrückung von Störanteilen.

Aus dem BCG-Signal können dann neben der I- und J-Wellenamplituden weitere diagnostisch relevante Parameter bestimmt werden, z.B.:

- ein Maß für das Herzzeitvolumen (cardiac output (CO)) aus dem Maß für das Schlagvolumen und der aus dem BCG, EKG, IEGM oder aus anderen Signalkomponenten des Beschleunigungssensors ermittelten Herzfrequenz

- ein Kontraktilitätsmaß des linken Ventrikels, beispielsweise aus dem Anstieg der J-Welle des BCG-Signals

- Maße für die elektromechanische Kopplung unter Einbeziehung des EKG/IEGM, z.B. Zeitintervall zwischen R-Zacke im EKG und dem Maximum der J-Welle im BCG (R-J-Zeit)
- unter Einbeziehung des Drucksignals eines (implantierten) Drucksensors: die jeweilige zeitliche Synchronisation des Drucksignals zum tatsächlichen Blutauswurf
- die Körperlage des Patienten unter Auswertung der Vektoren des BCG und des Gravitationsfeldes; und/oder
- die aktuellen Lage des Implantats im Körper aus dem Vektor des BCG (und zusätzlich ggf. des Vektors der Atmungsbeschleunigung), z.B. zur Ausrichtung von Ultraschallsensoren oder Telemetrieantennen

In Bezug auf die Bestimmung eines Maßes für das Herzzeitvolumen ist ein Gerät mit einer Herzratenerfassungseinheit bevorzugt, die ausgebildet ist, ein Herzratensignal durch Erfassen wiederkehrender Wellen in dem Ballistokardiogramm zu bestimmen. Das Herzratensignal kann aber auch auf an sich bekannte Weise aus einem Elektrokardiogramm durch Bestimmen der Frequenz, mit der beispielsweise R-Zacken wiederkehren, gewonnen werden. Das Herzzeitvolumen ergibt sich im Wesentlichen durch Multiplikation der Herzrate mit dem Schlagvolumen und kann dementsprechend aus dem Herzratensignal und dem das Schlagvolumen repräsentierenden Ausgangssignal der BCG-Auswerteeinheit gebildet werden.

Das implantierbare medizinische Gerät verfügt vorzugsweise über eine Telemetrieeinheit, die eine drahtlose Datenkommunikation mit einem externen Gerät (Patientengerät), das Telemonitoring, eine Kommunikation mit einem Servicecenter oder dergleichen erlaubt.

Dementsprechend können einige oder alle zu bestimmenden Parameter (siehe obige Auflistung) ganz oder teilweise im implantierbaren medizinischen Gerät, in einem Patientengerät oder einem Servicecenter bestimmt werden.

Aus den Parametern können Trends und Trendparameter abgeleitet werden.

Die so ermittelten Parameter oder Trendparameter werden gemäß bevorzugter Ausführungsvarianten der Erfindung weiterverarbeitet, um

- ein Monitoring des Status der Herzinsuffizienz durchzuführen;
- eine Einbindung in ein Vorhersagemodell zur Prognose der Krankheitsentwicklung oder der Therapiewirkung zu bewirken;
- eine Optimierung und Nachführung von Therapieparametern, z.B. bei Herzschrittmacher, ICD oder CRT-Geräten (insbesondere eine Optimierung und Nachführung von atrioventrikulärer Verzögerungszeit (AV-Delay) und interventrikulärer Verzögerungszeit (VV-Delay)) durchzuführen; und/oder
- ein Monitoring von Medikamentenwirkung oder -nebenwirkungen sowie eine Optimierung von Medikamententherapien zu unterstützen.

Wie weiter oben bereits ausgeführt, ist der Beschleunigungssensor dafür ausgebildet Beschleunigungen in drei Achsen zu erfassen und ein entsprechendes 3D-Akzelerometer-Ausgangssignal als Beschleunigungssignal an die BCG-Erfassungseinheit auszugeben. Die BCG-Erfassungseinheit ist ausgebildet, aus dem Beschleunigungssignal ein Ballistokardiogramm (BCG) abzuleiten, indem die BCG-Erfassungseinheit den in Körperlängsrichtung wirkenden Beschleunigungsvektor ermittelt und dessen zeitlichen Verlauf auswertet.

Die erfindugsgemässe BCG-Auswerteeinheit ist ausgebildet in einem von der BCG-Erfassungseinheit stammenden Ballistokardiogramm I- und J-Wellen zu bestimmen und aus der Amplitudendifferenz jeweils aufeinander folgender I- und J-Wellen das ein jeweiliges Schlagvolumen repräsentierende Ausgangssignal zu bilden.

Vorzugsweise weist das implantierbare medizinische Gerät eine Sensingeinheit mit angeschlossener oder anzuschießender Elektrode zur Aufnahme eines Elektrokardiogramms (EKGs) auf, die ausgebildet ist in dem aufgenommen EKG eine jeweilige Ventrikelkontraktion kennzeichnende R-Zacke zu detektieren, wobei das implantierbare medizinische Gerät außerdem eine mit der Sensingeinheit und der BCG-Auswerteeinheit verbundene Steuereinheit aufweist, die ausgebildet ist, eine Zeitdifferenz zwischen dem Zeitpunkt des Auftretens einer R-Zacke im Elektrokardiogramm und dem Zeitpunkt des Auftretens einer der R-Zacke zugeordneten J-Welle im Ballistokardiogramm als R-J-Zeit zu bestimmen.

Weitere bevorzugte Ausführungsvarianten ergeben sich durch weitere Kombination bevorzugter Merkmale miteinander sowie aus der nachfolgenden Beschreibung beispielhafter Ausführungsvarianten.

Die Erfindung soll nun anhand von Ausführungsbeispielen mit Bezug auf die Figuren näher erläutert werden. Von diesen zeigt:

Fig. 1    einen Einkammer-Kardioverter/Defibrillator mit angeschlossener Elektrodenleitung;

Fig. 2    einige Komponenten des Einkammer-Kardioverter/Defibrillators aus Figur 1 in einem schematischen Blockschaltbild;

Fig. 3    einen Dreikammer-Kardioverter/Defibrillator mit angeschlossenen Elektrodenleitungen;

Fig. 4    einige Komponenten des Dreikammer-Kardioverter/Defibrillators aus Figur 3 in einem schematischen Blockschaltbild;

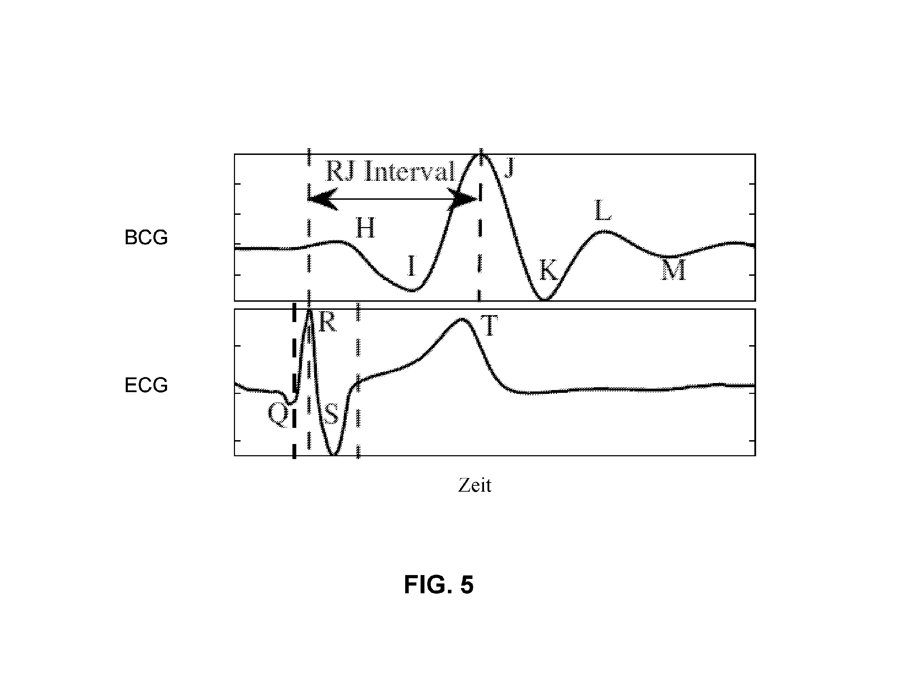

Fig. 5    Zusammenhänge zwischen Ballistokardiogramm, EKG, Herztönen und anderen mit dem Herzzyklus assoziierten Größen;

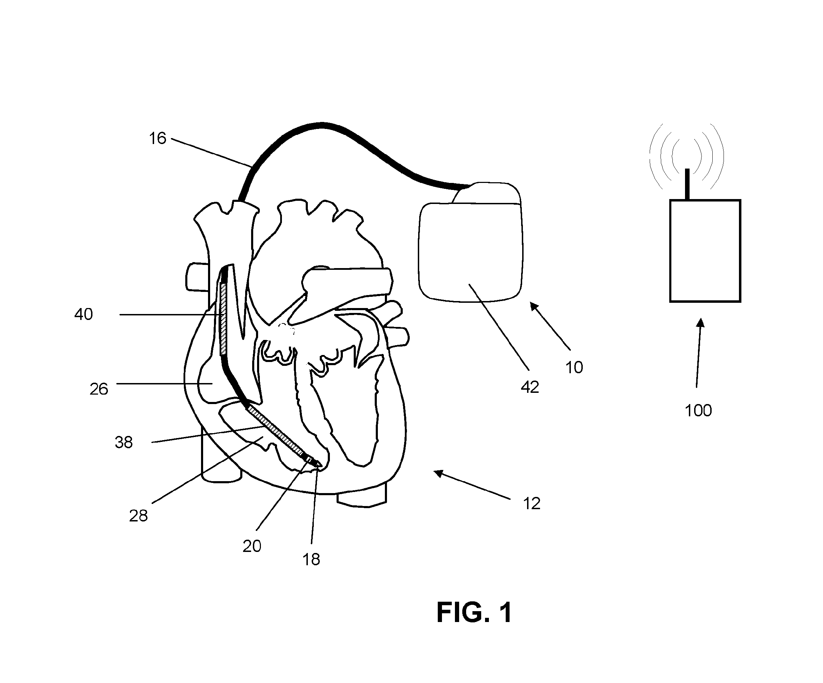

Figur 1 zeigt ein implantierbares medizinisches Gerät 10 in Form eines Einkammer-Kardioverter/Defibrillator (ICD) als Herzstimulator. An diesen ist eine einzige Elektrodenleitung angeschlossen, nämlich eine rechtsventrikuläre Elektrodenleitung 16. Im implantierten Zustand endet die rechtsventrikuläre Elektrodenleitung 16 im rechten Ventrikel 28 des Herzens 12.

Die rechtsventrikuläre Elektrodenleitung 16 trägt an ihrem distalen Ende eine rechtsventrikuläre Spitzenelektrode 18 und wenig entfernt davon eine rechtsventrikuläre Ringelektrode 20. Diese Elektroden dienen der Aufnahme elektrischer Potenziale im rechten Ventrikel sowie der Abgabe von Stimulationsimpulsen an den rechten Ventrikel im normalen Schrittmacherbetrieb.

Die rechtsventrikuläre Elektrodenleitung 16 trägt außerdem noch eine distale Schockelektrode RV Coil 38 und eine proximale Schockelektrode SVC Coil 40, die der Abgabe von Defibrillationsschocks im Falle ventrikulären Kammerflimmerns dienen, also im Falle einer (echten) ventrikulären Fibrillation. Die distale Schockelektrode 38 ist im rechten Ventrikel angeordnet und die proximale Schockelektrode 40 in der Superior Vena Cava (SVC).

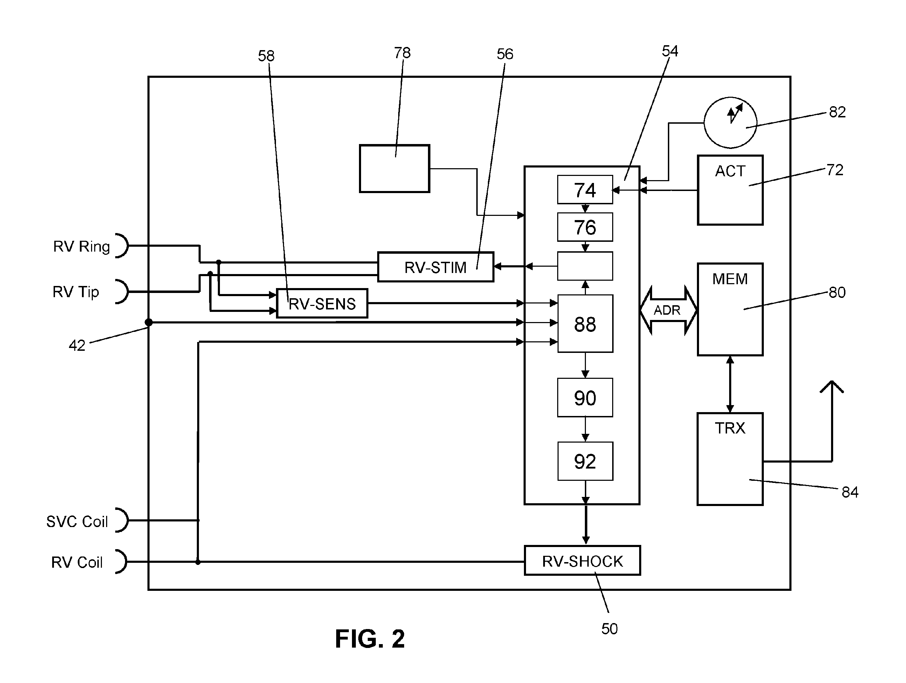

Figur 2 zeigt die Hauptbestandteile des Herzstimulators 10 aus Figur 1. Auf der linken Seite sind die elektrischen Anschlüsse für die verschiedenen Elektroden 18, 20, 38 und 40 dargestellt. Die Schockelektroden 38 und 40 sind mit einem rechtsventrikulären Schockimpulsgenerator 50 verbunden. Der Schockgenerator 50 ist mit einer Stimulationssteuereinheit 54 verbunden, die den Schockimpulsgenerator 50 bei Bedarf zur Erzeugung und Abgabe eines Defibrillationsschocks ansteuert. Weiterhin umfasst der Schockgenerator 50 in einer bevorzugten Ausführungsform in Fig. 2 nicht näher dargestellte Umschaltmittel, mittels derer entweder eine oder beide der Schockelektroden 38 und 40 und/oder das Gehäuse 42 mit dem Schockgenerator verbunden sind, so dass der Defibrillationsschock über eine beliebige Kombination der Schockelektroden 38 und 40 und des Gehäuses 42 abgegeben werden kann.

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensing-Einheit 58 verbunden. Sowohl die rechtsventrikuläre Stimulationseinheit 56 als auch die rechtsventrikuläre Sensing-Einheit 58 sind jeweils mit der Steuereinheit 54 verbunden.

Die rechtsventrikuläre Stimulationseinheit 56 ist dazu ausgebildet, auf ein Ansteuersignal der Steuereinheit 54 hin einen rechtsventrikulären Stimulationsimpuls zu erzeugen und im Anschluss für die rechtsventrikuläre Ringelektrode RV Ring 20 die rechts-ventrikuläre Spitzenelektrode RV Tip 18 abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Ringelektrode RV Tip und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten rechten Ventrikel 28 weiter aus und sorgt so für eine stimulierte Kontraktion des rechten Ventrikels 28.

Die rechtsventrikuläre Sensing-Einheit 58 ist dazu ausgebildet, an dem Anschluss für die rechtsventrikuläre Ringelektrode RV Ring 20 und die rechtsventrikuläre Tipelektrode RV Tip 18 anliegende elektrische Potenziale zunächst durch einen Eingangsverstärker zu verstärken und zu filtern. Weiterhin ist die rechtsventrikuläre Sensing-Einheit 58 ausgebildet, den Verlauf der an ihren Eingängen anliegenden elektrischen Signale derart auszuwerten, dass die rechtsventrikuläre Sensing-Einheit 58 selbsttätig eine natürliche (intrinsische), d.h. selbsttätige, Kontraktion des rechten Ventrikels 28 detektiert. Dies kann beispielsweise dadurch geschehen, dass der Verlauf des an den Eingängen der rechtsventrikulären Sensing-Einheit 58 anliegenden Signals mit einem Schwellenwert verglichen wird. Typischerweise ist die größte Amplitude des Signals in Form der sogenannten R-Zacke kennzeichnend für eine natürliche Kontraktion des rechten Ventrikels 28, die durch Schwellenwertvergleich detektiert werden kann. Die rechtsventrikuläre Sensing-Einheit 58 gibt daraufhin ein entsprechendes, eine natürliche Kontraktion des rechten Ventrikels 28 anzeigendes Ausgangssignal an die Steuereinheit 54 aus.

Zur Aufnahme intrakardialer Elektrokardiogramme weist die Steuereinheit 54 eine EKG-Erfassungseinheit 88 auf, die einerseits mit dem Gehäuse 42 des Herzstimulators 10 als einer ersten Elektrode und außerdem mit der ventrikulären Schockelektrode 38 als zweiter Elektrode verbunden ist. Die Elektrokardiogramm-Erfassungseinheit 88 ist außerdem mit der Sensing-Einheit 58 verbunden. Die Sensing-Einheit 58 generiert immer dann, wenn sie ein ventrikuläres Ereignis erfasst (siehe oben), ein ventrikuläres Markersignal. Die Elektrokardiogramm-Erfassungseinheit 88 nutzt dieses Markersignal, um in ihrem jeweils erfassten Elektrokardiogramm einen Signalabschnitt zu detektieren, der einen QRS-Komplex enthält.

In gleicher Art und Weise können auch für die Schockelektroden 38 und 40 eine oder mehrere (in Fig. 2 nicht dargestellte) Sensing-Einheiten vorgesehen sein. Diese Sensing-Einheit oder Sensing-Einheiten sind bevorzugt ausgebildet, Signale zwischen den Schockelektroden 38 und 40, zwischen den Schockelektrode 38 und Gehäuse 42 oder zwischen Schockelektrode 40 und Gehäuse 42 zu erfassen. Dies ist in Fig. 2 schematisch durch die Verbindung der Schockelektroden 38 und 40 sowie des Gehäuses 42 mit der Elektrokardiogramm-(EKG-) Erfassungseinheit 88 dargestellt.

Als weiterer Bestandteil des Herzstimulators 10 ist ein Beschleunigungssensor 72 mit der Stimulationssteuereinheit 54 verbunden und in das Gehäuse 42 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Steuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Der Beschleunigungssensor 72 ist ausgebildet, Beschleunigungen in drei Achsen zu erfassen und ein entsprechendes 3D-Akzelerometer-Ausgangssignal als Beschleunigungssignal an die Steuereinheit 54 auszugeben. Die Steuereinheit 54 besitzt eine BCG-Erfassungseinheit 74, die ausgebildet ist, das Beschleunigungssignal (also im konkreten Fall das 3D-Akzelerometer-Ausgangssignal) zu verarbeiten und aus dem 3D-Akzelerometer-Ausgangssignal ein Ballistokardiogramm (BCG) abzuleiten, indem die BCG-Erfassungseinheit den in Körperlängsrichtung wirkenden Beschleunigungsvektor ermittelt und dessen zeitlichen Verlauf auswertet.

Mit der BCG-Erfassungseinheit 74 verbunden ist eine BCG-Auswerteeinheit 76, die ausgebildet ist ein von der BCG-Erfassungseinheit 74 stammendes Ballistokardiogramm dahingehend auszuwerten, dass die BCG-Auswerteeinheit ein ein jeweiliges Schlagvolumen repräsentierendes Ausgangssignal liefert, indem die BCG-Auswerteeinheit 76 in einem von der BCG-Erfassungseinheit stammenden Ballistokardiogramm I- und J-Wellen bestimmt und aus der Amplitudendifferenz jeweils aufeinander folgender I- und J-Wellen oder der Amplitude der J-Welle das ein jeweiliges Schlagvolumen repräsentierende Ausgangssignal ableitet.

Die Steuereinheit 54 ist ausgebildet, eine Zeitdifferenz zwischen dem Zeitpunkt des Auftretens einer R-Zacke im Elektrokardiogramm - beispielsweise repräsentiert durch das entsprechende Markersignal - und dem Zeitpunkt des Auftretens einer der R-Zacke zugeordneten J-Welle im Ballistokardiogramm als R-J-Zeit zu bestimmen.

Aus den Signalen des Beschleunigungssensors kann somit die Aktivität des Patienten und dessen Körperlage ermittelt werden. Die Messung des Ballistogramms kann vorzugsweise zu Zeiten erfolgen, in denen der Patient in Ruhe ist und liegt, um den Lageeinfluss und Störungen durch Körperbewegungen zu verringern.

Der Beschleunigungssensor kann sich - so wie in Figur 2 dargestellt - im oder am Gehäuse eines IMD befinden oder von diesem abgesetzt sein und beispielsweise über ein Kabel mit dem IMD verbunden sein.

Weiterhin umfasst der Herzstimulator 10 eine Speichereinheit 80, die mit der Steuereinheit 54 verbunden ist und es erlaubt, von der Steuereinheit 54 erzeugte oder ausgewertete Signale zu speichern. Andererseits erlaubt es die Speichereinheit 80, Steuerprogramme für die Steuereinheit 54 in veränderbarer Form zu speichern. Des Weiteren ist die Steuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Insbesondere erlaubt es die Telemetrieeinheit 84, von dem Herzstimulator 10 erfasste Daten einschließlich eines jeweiligen Ballistokardiogramms oder daraus abgeleiteter Größen zusammen mit aktuell geltenden Therapieparametern via externes Gerät an ein zentrales Servicecenter zu übermitteln, wo diese Daten weiter ausgewertet und ggf. optimierte Therapieparameter ermittelt werden können. Näheres ist hierzu weiter unten ausgeführt.

Ein weiteres, optionales Merkmal des Herzstimulators 10 ist ein Drucksensor 78 zum Erfassen eines intrakardialen Drucks, der ebenfalls mit der Steuereinheit 54 verbunden ist.

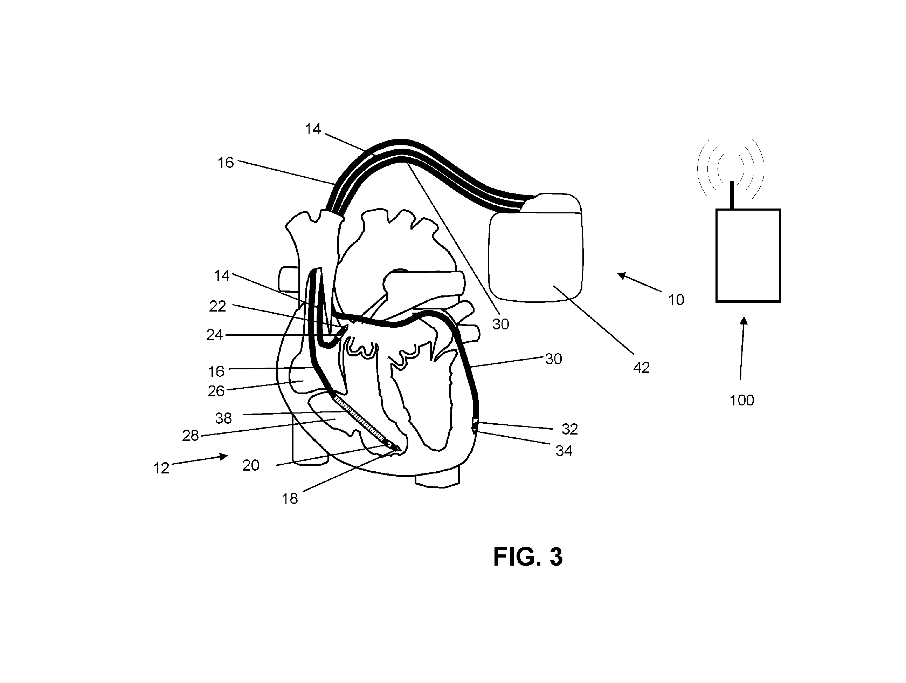

Figur 3 zeigt ein Implantat 10 in Form eines biventrikulären Dreikammer-Herzschrittmachers und Kardioverters/Defibrillators (ICD), der als CRT-Implantat für die kardiale Resynchronisationstherapie (CRT) eingesetzt werden kann. An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel des Herzens.

Die rechtsatriale Elektrodenleitung 14 trägt an ihrem distalen Ende eine rechtsatriale Spitzenelektrode 22 und in geringem Abstand davon eine rechtsatriale Ringelektrode 24. In ähnlicher Weise trägt die rechtsventrikuläre Elektrodenleitung 16 an ihrem distalen Ende eine rechtsventrikuläre Spitzenelektrode 18 und wenig entfernt davon eine rechtsventrikuläre Ringelektrode 20. Auch am distalen Ende der linksventrikulären Elektrodenleitung 30 ist eine linksventrikuläre Spitzenelektrode 34 und wenig entfernt davon eine linksventrikuläre Ringelektrode 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Diese braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel 38 als Defibrillationselektrode.

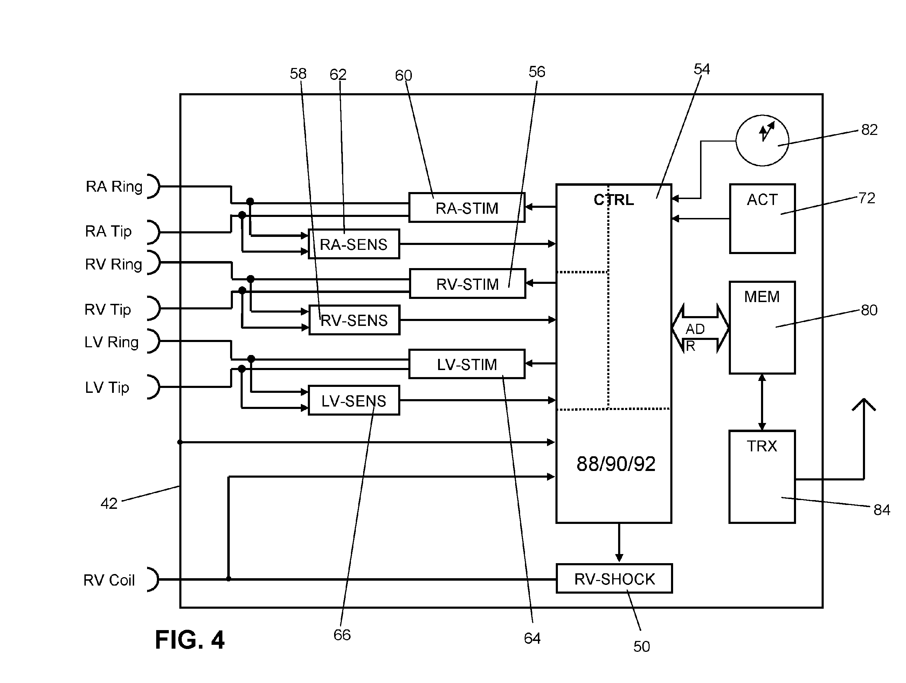

In Figur 4 sind die Hauptbestandteile des Herzstimulators 10 dargestellt. Ähnlich Figur 2 sind auf der linken Seite die elektrischen Anschlüsse für die verschiedenen Elektroden 18, 20, 22, 24, 32, 34 und 38 dargestellt. Die (einzige) Schockelektrode 38 ist mit einem rechtsventrikulären Schockimpulsgenerator 50 verbunden.

In Bezug auf die rechtsventrikuläre Spitzenelektrode RV Tip sowie die rechtsventrikuläre Ringelektrode RV Ring, das Gehäuse 42 (als Neutralelektrode), die rechtsventrikuläre Stimulationseinheit 56, die rechtsventrikuläre Sensing-Einheit 58 und die Steuereinheit 54 gilt entsprechend das oben zu Figur 2 Gesagte.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensing-Einheit 62 verbunden, die jeweils ihrerseits wiederum mit der Steuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechtsventrikulären Stimulationsimpulse. Die rechtsatriale Sensing-Einheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 26 kennzeichnet. Detektiert die rechtsatriale Sensing-Einheit 62 eine entsprechende P-Welle, erzeugt sie ein Ausgangssignal und gibt dieses an die Steuereinheit 54 weiter, welches eine natürliche Kontraktion des rechten Atriums 26 kennzeichnet.

In gleicher Weise sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensing-Einheit 66 verbunden. Die linksventrikuläre Stimulationseinheit 64 und die linksventrikuläre Sensing-Einheit 66 sind ebenso mit der Steuereinheit 54 verbunden. Beide funktionieren ähnlich wie die bereits beschriebenen Stimulationseinheiten 56 und 60 und Sensing-Einheiten 58 und 62.

In gleicher Art und Weise kann auch für die Schockelektrode 38 eine (in Figur 4 nicht dargestellte) Sensing-Einheit vorgesehen sein. Diese Sensing-Einheit ist bevorzugt ausgebildet, Signale zwischen Schockelektrode 38 und Gehäuse 42 zu erfassen. Dies ist in Figur 4 schematisch durch die Verbindung der Schockelektrode 38 sowie des Gehäuses 42 mit der EKG Erfassungseinheit 88 dargestellt.

Nachfolgend wird anhand spezieller Ausführungsbeispiele die Erfassung und Auswertung von Ballistokardiogrammen durch entsprechende implantierbare medizinische Geräte erläutert:

Ausführungsbeispiel A

Ein CRT-Implantat (siehe Figur 4) als implantierbares medizinisches Gerät 10 enthält einen 3-achsigen Beschleunigungssensor 72. Aus den Signalen des Beschleunigungssensors 72 wird nach obigem Verfahren das Ballistokardiogramm bestimmt. Aus dem Ballistokardiogramm, z.B. aus der Amplitudendifferenz der I- und J-Welle, wird ein Maß für das Schlagvolumen ermittelt und zusätzlich mit Hilfe des IEGM die R-J-Zeit bestimmt. Anhand der R-J-Zeit und des Maßes für das Schlagvolumen werden vom implantierbaren medizinischen Gerät 10 automatisch die Therapieparameter optimiert und den physiologischen Erfordernissen nachgeführt. Die Maße für das Schlagvolumen, die R-J-Zeiten und die vom implantierbaren medizinischen Gerät 10 eingestellten Therapieparameter werden im implantierbaren medizinischen Gerät 10 gespeichert und über eine Telemetrieverbindung an ein Homemonitoring-Servicecenter übertragen. Im Servicecenter werden aus den übertragenen Parametern und Therapieparametern Trends generiert und sie werden als Zusatzgrößen in einem Vorhersagemodell (Prädiktor) verarbeitet. Die Parameter und die Therapieparameter können auch mit Schwellwerten verglichen werden, um nötigenfalls eine Alarmmeldung zu generieren.

Ausführungsbeispiel B

Ein Monitoring-Implantat als implantierbares medizinisches Gerät ohne eigene Therapiefunktion enthält neben anderen Sensoren, z.B. für das EKG, einen 3-Achs-Beschleunigungssensor, der auch zur Bestimmung der Patientenaktivität verwendet wird. Aus den Signalen des Beschleunigungssensors wird nach obigem Verfahren zusätzlich das Ballistokardiogramm bestimmt. Aus dem Ballistokardiogramm, z.B. aus der Amplitude der J-Welle, wird ein Maß für das Schlagvolumen ermittelt und gemeinsam mit den anderen Sensorwerten über eine Telemetrieverbindung an ein externes Gerät (Patientengerät) und ein Homemonitoring-Servicecenter übertragen. Im Homemonitoring-Servicecenter wird das Schlagvolumen in die Beurteilung des Gesundheitszustandes des Patienten mit einbezogen. Auch können damit Therapiewirkungen, z.B. Medikamentierungen, überwacht und die Therapie gegebenenfalls angepasst werden.

Kombination mit anderen Sensorgrößen

Die Berechnung und Auswertung des Ballistokardiogramm kann durch weitere Sensorgrößen ergänzt werden. Diese dienen z.B. als Triggersignal für eine herzzyklussynchrone Mittelung des Beschleunigungssignals oder um die aus Ballistokardiogramm berechnete Kenngröße einem bestimmten physiologischen Zustand zuzuordnen, z.B. Ruhe, Aktivität etc.

Gleichzeitig können die aus dem Ballistokardiogramm bestimmten Kenngrößen, insbesondere Zeitkonstanten, als Zusatzinformationen bei der Auswertung anderer Messgrößen verwendet werden, z.B. für Impedanz oder Blutdruck.

**Patentansprüche**

1. Implantierbares medizinisches Gerät mit einem integrierten oder anzuschließenden, ebenfalls implantierbaren Beschleunigungssensor, der ausgebildet ist, Beschleunigungen in drei Achsen zu erfassen und ein entsprechendes 3D-Akzelerometer-Ausgangssignal als Beschleunigungssignal auszugeben und einer mit dem Beschleunigungssensor verbundenen oder zu verbindenden BCG-Erfassungseinheit, die ausgebildet ist, ein von dem Beschleunigungssensor stammendes Beschleunigungssignal zu verarbeiten und aus dem Akzelerometer-Ausgangssignal ein Ballistokardiogramm (BCG) abzuleiten indem die BCG-Erfassungseinheit den in Körperlängsrichtung wirkenden Beschleunigungsvektor ermittelt und dessen zeitlichen Verlauf auswertet, sowie mit einer mit der BCG-Erfassungseinheit verbundenen BCG-Auswerteeinheit, die ausgebildet ist, ein von der BCG-Erfassungseinheit stammendes Ballistokardiogramm dahingehend auszuwerten, dass die BCG-Auswerteeinheit ein ein jeweiliges Schlagvolumen repräsentierendes Ausgangssignal liefert, **dadurch gekennzeichnet dass** die BCG-Auswerteeinheit ausgebildet ist, in einem von der BCG-Erfassungseinheit stammenden Ballistokardiogramm I- und J-Wellen zu bestimmen und aus der Amplitudendifferenz jeweils aufeinander folgender I- und J-Wellen das ein jeweiliges Schlagvolumen repräsentierende Ausgangssignal zu bilden.

2. Implantierbares medizinisches Gerät gemäß Anspruch 1, **dadurch gekennzeichnet, dass** das implantierbare medizinische Gerät eine Sensingeinheit mit angeschlossener oder anzuschießender Elektrode zur Aufnahme eines Elektrokardiogramms (EKGs) aufweist, die ausgebildet ist in dem aufgenommen EKG eine jeweilige Ventrikelkontraktion kennzeichnende R-Zacke zu detektieren, wobei das implantierbare medizinische Gerät außerdem eine mit der Sensingeinheit und der BCG-Auswerteeinheit verbundene Steuereinheit aufweist, die ausgebildet ist, eine Zeitdifferenz zwischen dem Zeitpunkt des Auftretens einer R-Zacke im Elektrokardiogramm und dem Zeitpunkt des Auftretens einer der R-Zacke zugeordneten J-Welle im Ballistokardiogramm als R-J-Zeit zu bestimmen.

3. Implantierbares medizinisches Gerät gemäß einem der Ansprüche 1 bis 2, **dadurch gekennzeichnet, dass** das implantierbare medizinische Gerät eine Herzratenerfassungseinheit aufweist, die ein die Herzrate repräsentierendes Herzratensignal liefert, sowie eine mit der Herzratenerfassungseinheit und der BCG-Auswerteeinheit verbundene Bestimmungseinheit zum Bestimmen eines Herzzeitvolumens, die ausgebildet ist, auf Basis des Herzratensignals und des ein jeweiliges Schlagvolumen repräsentierendes Ausgangssignals ein ein jeweiliges Herzzeitvolumen repräsentierendes Ausgangssignal zu generieren.

4. Implantierbares medizinisches Gerät gemäß Anspruch 3, **dadurch gekennzeichnet, dass** die Herzratenerfassungseinheit ausgebildet ist, das Herzratensignal durch Erfassen wiederkehrender Wellen in dem Ballistokardiogramm zu bestimmen.

5. Implantierbares medizinisches Gerät gemäß einem der Ansprüche 1 bis 4, **dadurch gekennzeichnet, dass** die BCG-Auswerteeinheit ausgebildet ist, ein Kontraktilitätsmaß für einen linken Ventrikel aus dem Ballistokardiogramm abzuleiten, und zwar vorzugsweise, indem die BCG-Auswerteeinheit dazu ausgebildet ist, in einem von der BCG-Erfassungseinheit stammenden Ballistokardiogramm eine jeweilige J-Welle zu bestimmen und das Kontraktilitätsmaß aus dem Anstieg der J-Welle abzuleiten.

6. Implantierbares medizinisches Gerät gemäß einem der Ansprüche 1 bis 5, **dadurch gekennzeichnet, dass** das implantierbare medizinische Gerät einen Drucksensor aufweist oder mit einem implantierbaren Drucksensor zu verbinden ist und eine Auswerteeinheit aufweist, die ausgebildet ist eine jeweilige zeitliche Synchronisation des Drucksignals zum tatsächlichen Blutauswurf zu erfassen.

7. Implantierbares medizinisches Gerät gemäß einem der Ansprüche 1 bis 6, **dadurch gekennzeichnet, dass** die BCG-Auswerteeinheit ausgebildet ist eine Körperlage eines Patienten unter Auswertung der Richtung des Vektors des Ballistokardiogramms und der Richtung des Gravitationsfeldes zu bestimmen.

8. Implantierbares medizinisches Gerät gemäß einem der Ansprüche 1 bis 7, **dadurch gekennzeichnet, dass** die BCG-Auswerteeinheit ausgebildet ist eine aktuellen Lage des implantierbaren medizinischen Gerätes in einem Körper eines Patienten durch Auswerten der Richtung des Vektors des Ballistokardiogramms und vorzugsweise zusätzlich der Richtung des Vektors der Atmungsbeschleunigung zu bestimmen.

9. Implantierbares medizinisches Gerät gemäß einem der Ansprüche 1 bis 8, **dadurch gekennzeichnet, dass** das implantierbare medizinische Gerät eine Telemetrieeinheit zur drahtlosen Datenübertragung aufweist und die BCG-Auswerteeinheit Bestandteil eines telemetrisch mit dem implantierbaren medizinischen Gerät zu verbindenden externen Gerätes ist.

**10.** Implantierbares medizinisches Gerät gemäß einem der Ansprüche 1 bis 9, **dadurch gekennzeichnet, dass** das implantierbare medizinische Gerät als biventrikulärer Herzstimulator für eine kardiale Resynchronisationstherapie ausgebildet ist die BCG-Auswerteeinheit mit einer Therapiesteuereinheit zur Optimierung einer atrioventrikulären und/oder einer interventrikulären Verzögerungszeit verbunden ist.

**11.** Implantierbares medizinisches Gerät gemäß einem der Ansprüche 1 bis 10, **dadurch gekennzeichnet, dass** das implantierbare medizinische Gerät als Monitoring-Implantat ohne eigene Therapiefunktion ausgebildet ist.

**Claims**

**1.** An implantable medical device comprising an acceleration sensor, which is integrated or is to be connected, likewise is implantable, and is configured to capture accelerations in three axes and to output a corresponding 3D accelerometer output signal as acceleration signal, and comprising a BCG capturing unit, which is connected or is to be connected to the acceleration sensor and is configured to process an acceleration signal originating from the acceleration sensor and to derive a ballistocardiogram (BCG) from the accelerometer output signal in that the BCG capturing unit determines the acceleration vector acting in the longitudinal direction of a body and evaluates the time curve thereof, and also comprising a BCG evaluation unit, which is connected to the BCG capturing unit and is configured to evaluate a ballistocardiogram originating from the BCG capturing unit such that the BCG evaluation unit delivers an output signal representing a respective stroke volume, **characterised in that** the BCG evaluation unit is configured to determine I-waves and J-waves in a ballistocardiogram originating from the BCG capturing unit and to form the output signal representing a respective stroke volume on the basis of the amplitude difference between successive I-waves and J-waves.

**2.** The implantable medical device according to Claim 1, **characterised in that** the implantable medical device has a sensing unit with an electrode that is connected or is to be connected for recording an electrocardiogram (ECG), which sensing unit is configured to detect an R-wave characterising a respective ventricular contraction in the recorded ECG, wherein the implantable medical device additionally has a control unit connected to the sensing unit and the BCG evaluation unit, which control unit is configured to determine, as R-J interval, a time difference between the time at which an R-wave occurs in the electrocardiogram, and the time at which a J-wave associated with the R-wave occurs in the ballistocardiogram.

**3.** The implantable medical device according to one of Claims 1 to 2, **characterised in that** the implantable medical device has a heart rate capturing unit, which delivers a heart rate signal representing the heart rate, and also has a determining unit, which is connected to the heart rate capturing unit and the BCG evaluation unit, for determining a cardiac output, which determining unit is configured to generate an output signal representing a respective cardiac output on the basis of the heart rate signal and the output signal representing a respective stroke volume.

**4.** The implantable medical device according to Claim 3, **characterised in that** the heart rate capturing unit is configured to determine the heart rate signal by capturing recurring waves in the ballistocardiogram.

**5.** The implantable medical device according to one of Claims 1 to 4, **characterised in that** the BCG evaluation unit is configured to derive a contractility measure for a left ventricle from the ballistocardiogram, preferably **in that** the BCG evaluation unit is configured to determine a respective J-wave in a ballistocardiogram originating from the BCG capturing unit and to derive the contractility measure from the rise of the J-wave.

**6.** The implantable medical device according to one of Claims 1 to 5, **characterised in that** the implantable medical device has a pressure sensor or is to be connected to an implantable pressure sensor, and has an evaluation unit configured to capture a respective temporal synchronisation of the pressure signal to the actual blood ejection.

**7.** The implantable medical device according to one of Claims 1 to 6, **characterized in that** the BCG evaluation unit is configured to determine a patient body position on the basis of evaluation of the direction of the vector of the ballistocardiogram and the direction of the gravitational field.

**8.** The implantable medical device according to one of Claims 1 to 7, **characterised in that** the BCG evaluation unit is configured to determine a current position of the implantable medical device in a body of a patient by evaluating the direction of the vector of the ballistocardiogram and preferably additionally the direction of the vector of the respiratory acceleration.

9. The implantable medical device according to one of Claims 1 to 8, **characterised in that** the implantable medical device has a telemetry unit for wireless data transmission, and the BCG evaluation unit is part of an external device to be placed in telemetric communication with the implantable medical device.

10. The implantable medical device according to one of Claims 1 to 9, **characterised in that** the implantable medical device is configured as a biventricular cardiac stimulator for cardiac resynchronization therapy, and the BCG evaluation unit is connected to a treatment control unit for optimising atrioventricular and/or interventricular delay.

11. The implantable medical device according to one of Claims 1 to 10, **characterised in that** the implantable medical device is configured as a monitoring implant without own treatment function.


**Revendications**

1. Appareil médical implantable avec un capteur d'accélération également implantable, intégré ou à raccorder, lequel est conçu pour détecter des accélérations sur trois axes et délivrer un signal de sortie d'accéléromètre 3D correspondant, en tant que signal d'accélération, et avec une unité de détection BCG reliée ou destinée à être reliée au capteur d'accélération, laquelle est conçue pour traiter un signal d'accélération provenant du capteur d'accélération et pour dériver un ballistocardiogramme (BCG) à partir du signal de sortie d'accéléromètre, l'unité de détection BCG détectant le vecteur d'accélération agissant dans le sens longitudinal du corps et évaluant la progression dans le temps de celui-ci, et avec une unité d'évaluation BCG reliée à l'unité de détection BCG, laquelle est conçue pour évaluer un ballistocardiogramme provenant de l'unité de détection BCG de telle sorte l'unité d'évaluation BCG délivre un signal de sortie représentant un volume systolique respectif, **caractérisé en ce que** l'unité d'évaluation est conçue pour déterminer des ondes I et J dans un ballistocardiogramme provenant de l'unité de détection BCG, et pour former le signal de sortie représentant un volume systolique respectif à partir de la différence d'amplitude d'ondes I et J respectivement consécutives.

2. Appareil médical implantable selon la revendication 1, **caractérisé en ce que** l'appareil médical implantable comporte une unité de détection avec une électrode raccordée ou destinée à être raccordée, pour l'enregistrement d'un électrocardiogramme (ECG), laquelle est conçue pour détecter un pic R caractérisant une contraction ventriculaire respective dans l'ECG enregistré, l'appareil médical implantable comportant en outre une unité de commande reliée à l'unité de détection et à l'unité d'évaluation BCG, laquelle est conçue pour déterminer une différence de temps entre le moment de l'apparition d'un pic R dans l'électrocardiogramme et le moment de l'apparition d'une onde J attribuée au pic R dans le ballistocardiogramme, en tant que temps R-J.

3. Appareil médical implantable selon l'une des revendications 1 à 2, **caractérisé en ce que** l'appareil médical implantable comporte une unité de détection du rythme cardiaque délivrant un signal de rythme cardiaque représentant le rythme cardiaque, ainsi qu'une unité de détermination reliée à l'unité de détection de rythme cardiaque et à l'unité d'évaluation BCG, pour la détermination d'un débit cardiaque, laquelle est conçue pour générer un signal de sortie représentant un débit cardiaque respectif, sur la base du signal de rythme cardiaque et du signal de sortie représentant un volume systolique respectif.

4. Appareil médical implantable selon la revendication 3, **caractérisé en ce que** l'unité de détection BCG de rythme cardiaque est conçue pour déterminer le signal de rythme cardiaque en détectant des ondes récurrentes dans le ballistocardiogramme.

5. Appareil médical implantable selon l'une des revendications 1 à 4, **caractérisé en ce que** l'unité d'évaluation BCG est conçue pour dériver une mesure de contractilité pour un ventricule gauche à partir du ballistocardiogramme, notamment de préférence de manière à ce que l'unité d'évaluation BCG soit conçue pour déterminer une onde J respective dans un ballistocardiogramme provenant de l'unité de détection BCG, et pour dériver la mesure de contractilité à partir de l'augmentation de l'onde J.

6. Appareil médical implantable selon l'une des revendications 1 à 5, **caractérisé en ce que** l'appareil médical implantable comporte un capteur de pression ou est destiné à être relié à un capteur de pression implantable et comporte une unité d'évaluation conçue pour détecter une synchronisation temporelle respective du signal de pression avec le débit sanguin réel.

7. Appareil médical implantable selon l'une des revendications 1 à 6, **caractérisé en ce que** l'unité d'évaluation BCG est conçue pour déterminer une position corporelle d'un patient en évaluant la direction du vecteur du ballistocardiogramme et la direction du champ gravitationnel.

**8.** Appareil médical implantable selon l'une des revendications 1 à 7, **caractérisé en ce que** l'unité d'évaluation BCG est conçue pour déterminer une position actuelle de l'appareil médical implantable dans un corps d'un patient en évaluant la direction du vecteur du ballistocardiogramme et de préférence également la direction du vecteur de l'accélération respiratoire.

**9.** Appareil médical implantable selon l'une des revendications 1 à 8, **caractérisé en ce que** l'appareil médical implantable comporte une unité de télémétrie pour la transmission de données sans fil, et l'unité d'évaluation BCG fait partie d'un appareil externe à relier par télémétrie à l'appareil médical implantable.

**10.** Appareil médical implantable selon l'une des revendications 1 à 9, **caractérisé en ce que** l'appareil médical implantable est conçu comme un stimulateur cardiaque biventriculaire pour une thérapie de resynchronisation cardiaque, et l'unité d'évaluation BCG est reliée à une unité de commande thérapeutique pour l'optimisation d'un temps de retard atrio-ventriculaire et/ou inter-ventriculaire.

**11.** Appareil médical implantable selon l'une des revendications 1 à 10, **caractérisé en ce que** l'appareil médical implantable est conçu comme un implant de monitoring sans fonction thérapeutique propre.

**FIG. 1**

FIG. 2

FIG. 3

EP 2 433 565 B1

FIG. 4

**FIG. 5**

# EP 2 433 565 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7395114 B
- EP 1762270 A
- US 7702389 B
- US 7139609 B
- US 7585279 B
- US 6978184 B
- US 20090138060 A1
- US 7660632 B

**In der Beschreibung aufgeführte Nicht-Patentliteratur**

- **M. ETEMADI ; O. T. INAN ; R. M. WIARD ; G. T. A. KOVACS ; L. GIOVANGRANDI.** Non-invasive assessment of cardiac contractility on a weighing scale. *IEEE EMBC 2009,* 2009, 6773-6776